# EUROPEAN PATENT APPLICATION

(11) **EP 4 710 899 A1**
(43) Date of publication of application: **18.03.2026**
(21) Application number: 24803677.4
(22) Date of filing: 07.05.2024
(51) Int. Cl.: A61F 7/00, A61N 1/36

(54) **COMPLEX STIMULATION DEVICE FOR PAIN MANAGEMENT AND PHYSICAL THERAPY**

(30) Priority: 09.05.2023 KR 20230059898
(71) Applicant: SJ Global Co., Ltd, Bucheon-si, Gyeonggi-do 14559 (KR)
(72) Inventor: HWANG, Yu An, Bucheon-si Gyeonggi-do 14559 (KR); JEON, Hyang Hee, Seoul 02622 (KR); SHIN, Hyun A, Seoul 07593 (KR); WANG, Geon Yeong, Bucheon-si Gyeonggi-do 14539 (KR); CHOI, Hoon Seong, Uiwang-si Gyeonggi-do 16028 (KR)
(74) Representative: Plougmann Vingtoft a/s
(86) International application number: PCT/KR2024/006105
(87) International publication number: WO 2024/232624

(57) **Abstract**

A multi-stimulation device for pain management and physical therapy according to an embodiment of the present invention includes: a first electrode having a predetermined surface area exposed to the outside to form a stimulation surface; a thermoelectric element in which, when one surface absorbs or generates heat by applied electric current, the other surface generates or absorbs heat, and one surface is brought into contact with a rear surface of the first electrode to provide cold or heat; a first heat dissipation plate being in contact with the other surface of the thermoelectric element and having a block shape with a predetermined thickness; a second heat dissipation plate coupled to the first heat dissipation plate and including a fin array having a surface area for dissipating heat into the atmosphere; a fan configured to generate a flow of air to the second heat dissipation plate; and a control panel configured to control the thermoelectric element or the first electrode, thereby controlling medium-frequency stimulation of the first electrode or heat absorption or heat generation of the thermoelectric element and forming the cold or heat stimulation on the stimulation surface, in which the second heat dissipation plate is arranged such that, when the stimulation surface faces a front surface of the device, fins of the fin array protrude toward a rear surface of the device.

## Description

### CROSS-REFERENCE TO RELATED APPLICATIONS

This U.S. non-provisional patent application claims priority under 35 U.S.C. § 119 of Korean Patent Application No. 10-2023-0059898, filed on May 09, 2023, the entire contents of which are hereby incorporated by reference.

### TECHNICAL FIELD

The present invention relates to a multi-stimulation device for pain management and physical therapy, and more particularly, to a multi-stimulation device having a portable handpiece shape to provide medical cold stimulation, heat stimulation, and medium-frequency stimulation, thereby being used for various indications.

### BACKGROUND ART

In general, heat and inflammation generated in a lesion (treatment area) destroy a cell and delay a treatment process. Typically, a medical device used for management and treatment of the lesion includes a cold stimulator and a hot stimulator.

The cold stimulator contracts a blood vessel in a limited portion to temporarily reduce a size of a blood cell, and this blood vessel contraction decreases an amount of blood flowing through the lesion. Cold stimulation may temporarily suppress nerve transmission to alleviate pain and minimize cell destruction in a wounded area to reduce swelling and inflammation.

Also, the cold stimulation has an advantage of smoothly absorbing drugs. It is disclosed in the paper titled "FARMACOTERAPIA A LOCALIZZAZIONE MIRATA MEDIANTE 'CRIOELETTROFORESI' NELLE PATOLOGIE ARTICOLARI E MUSCOLARI", published by A. Aloisi, M. Matera, and R. Potenza in 2002 that the cold stimulation is more effective than electrical stimulation in terms of drug absorption. According to the paper, when a temperature is maintained between -5°C and 0°C using ice or the like, drugs are most efficiently absorbed. Indications for cryotherapy by cold stimulation include acute contusions, muscle spasms, muscle stiffness, tachycardia, functional cardiac arrhythmias, myofascial pain, fascial disorders, sprains, myalgia, pain relief, and reduced bleeding.

On the other hand, the hot stimulator performs treatment by applying hot stimulation to a wounded area. The hot stimulator applies hot stimulation to an area experiencing mild pain such as muscular pain or joint pain to provide a hot pack effect and improve blood circulation. Indications for hot pack treatment by hot stimulation include increased blood flow, muscle relaxation, enhanced metabolism, pain relief, and improved tissue viscoelasticity.

The cold and hot stimulation may be used not only for medical purposes but also for skin care. The cold and hot stimulation supplies cold or heat to skin to increase stimulation and provides body balance and fatigue alleviation to manage clear and elastic skin.

A device for generating the above-described cold and hot stimulation requires an element, i.e., a thermoelectric element. Among the thermoelectric elements, a Peltier element uses the Peltier effect that is a phenomenon in which heat is absorbed or generated by electric current. Typically, a water-cooling device is used for the Peltier element to lower a temperature of a ceramic element to be less than zero. The water-cooling device has a disadvantage of increased device complexity and manufacturing costs because the device requires an additional device for cooling water although the water-cooling device has a high cooling efficiency. Also, the water-cooling device has inconvenience due to necessity of separately managing a coolant and a risk such as water leakage or freezing. Furthermore, the water-cooling device using the Peltier element has a disadvantage of requiring an increased volume of the stimulation device to achieve a high efficiency.

In order to solve the above-described limitations, the present applicant has disclosed an air-cooled apparatus capable of providing thermotherapy stimulations in Korean Patent Registration No. 1193935. The present applicant disclosed a stimulation apparatus capable of performing all of cold stimulation and heat stimulation in an air-cooling method by configuring a heat exchanger including a thermoelectric element, a heat block, a heat pipe, and a heat fin through the prior patent.

However, the prior patent of the present applicant has a limitation in that, since the heat fin requires a sufficient size in a longitudinal direction due to characteristics of the air-cooled heat exchanger, a head part of the product has a large volume, and a user feels inconvenience due to a poor center of gravity. Also, since it is difficult to mount a separate battery and charging module onto a handpiece due to a volume occupied by the heat exchanger, the device requires a power cable during use, which causes inconvenience to the user.

On the other hand, medium-frequency stimulation is frequently used as a physical therapy method for stimulating and relaxing a muscle by applying electrical medium-frequency current in addition to temperature-based therapy using cold and hot stimulation. The medium-frequency stimulation transmits the medium-frequency current to the muscle through an electrode to obtain an effect of stimulating and relaxing the muscle. The medium-frequency current physically stimulates the muscle to accelerate blood circulation in the muscle and reduce inflammation. Indications for medium-frequency therapy include pain control, muscle contraction/relaxation, blood flow increase, absorption of edema or hematoma, reduction in inflammation, improvement of wound healing, and improvement of urination.

As described above, the indications and effects of the cold and hot stimulation may produce a synergistic effect with those of the medium-frequency therapy. Since the multi-stimulation of the cold and hot stimulation and the medium-frequency stimulation may significantly improve muscle pain relief and recovery rate, the multi-stimulation may be particularly effective for pain alleviation and physical therapy for athletes.

The present applicant has devised a multi-stimulation device that is implemented as a battery-mounted rechargeable handpiece by improving the air-cooled structure, minimizing a volume of the device while securing a sufficient thermal conductivity performance, and minimizing a volume occupied by a heat block and a board while adding a medium-frequency electrode structure to a stimulation unit.

### [Related art document]

### [Patent document]

Korean Patent Registration No. 1193935

### DISCLOSURE OF THE INVENTION

### TECHNICAL PROBLEM

The present invention provides a multi-stimulation device capable of generating cold and hot stimulation in an air cooling method and simultaneously transmitting medium-frequency stimulation in combination with the cold and hot stimulation.

### TECHNICAL SOLUTION

An embodiment of the present invention provides a multi-stimulation device for pain management and physical therapy, the multi-stimulation device including: a first electrode having a predetermined surface area exposed to the outside to form a stimulation surface; a thermoelectric element in which, when one surface absorbs or generates heat by applied electric current, the other surface generates or absorbs heat, and one surface is brought into contact with a rear surface of the first electrode to provide cold or heat; a first heat dissipation plate being in contact with the other surface of the thermoelectric element and having a block shape with a predetermined thickness; a second heat dissipation plate coupled to the first heat dissipation plate and including a fin array having a surface area for dissipating heat into the atmosphere; a fan configured to generate a flow of air to the second heat dissipation plate; and a control panel configured to control the thermoelectric element or the first electrode, thereby controlling medium-frequency stimulation of the first electrode or heat absorption or heat generation of the thermoelectric element and forming the cold or heat stimulation on the stimulation surface, in which the second heat dissipation plate is arranged such that, when the stimulation surface faces a front surface of the device, fins of the fin array protrude toward a rear surface of the device.

In an embodiment, when the stimulation surface faces the front surface, the first electrode, the thermoelectric element, the first heat dissipation plate, the second heat dissipation plate, and the fan may be sequentially arranged on the same axis as the stimulation surface.

In an embodiment, a compound layer may be disposed on a contact surface in which the first heat dissipation plate and the second heat dissipation plate are coupled.

In an embodiment, a compound layer may be disposed on a contact surface in which the first electrode and the thermoelectric element are coupled.

In an embodiment, the compound layer that is formed by applying a viscous liquid may include thermal grease, thermally conductive paste, thermally conductive silicon, or a thermally conductive composite.

In an embodiment, the multi-stimulation device may further include a second electrode spaced a distance from an outer circumferential surface of the first electrode, in which the second electrode may be brought into contact with skin of a human body to serve as a ground electrode, and the stimulation surface may be formed by the first electrode and the second electrode.

In an embodiment, the first electrode, the thermoelectric element, the first heat dissipation plate, the second heat dissipation plate, and the fan may be disposed in the stimulation unit that constitutes a head of the housing, the control panel may be disposed in the handle unit of the housing, and the handle unit may include a rechargeable battery configured to supply power to the control panel.

In an embodiment, the handle unit may be divided into a first section and a second section, the control panel may be disposed in the first section, and the battery may be disposed in the second section.

In an embodiment, the control panel may have a stacked structure in which a first control panel and a second control panel are stacked and be disposed in the first section.

### ADVANTAGEOUS EFFECTS

According to the present invention, the heat dissipation module includes the first heat dissipation plate having the block shape to effectively absorbs and stores the heat applied to the thermoelectric element and the second heat dissipation plate having the fin array for heat dissipation. The first heat dissipation plate is formed by the metallic member with the minimized surface area and the predetermined thickness to have the high heat capacity, thereby receiving and storing the heat of the thermoelectric element to prevent the heat from being accumulated in thermoelectric element. The second heat dissipation plate receives the heat stored in the first heat dissipation plate and dissipates the received heat into the air. The second heat dissipation plate has the structure with the large surface area. The present invention may implement the multi-stimulation device as the compact handpiece by improving the air-cooling efficiency of the thermoelectric element using the two types of coupled heat dissipation plates and minimizing the volume of the second heat dissipation plate through the improvement of the cooling efficiency to prevent elongation of the stimulation unit.

Also, according to the present invention, the compound layer is formed on the coupling surface in which the two types of heat dissipation plates are coupled to improve the efficiency of the thermal conductivity. It is theoretically impossible for the two metal surfaces to be in complete contact and coupled with each other when the two types of metal heat dissipation plates are coupled by using the screw. The compound layer that is the viscous liquid improving the thermal conductivity may form the layer on the coupling surface to cause the effect of the complete contact and coupling between the first heat dissipation plate and the second heat dissipation plate and significantly improve the efficiency of the thermal conductivity from the first heat dissipation plate to the second heat dissipation plate.

Also, according to the present invention, as the first electrode and the second electrode are formed together on the stimulation surface, and the ground electrode is brought into contact with the skin, the leakage current may be minimized during the medium-frequency stimulation, the stimulation efficiency may be improved, and the user convenience of using the product may be secured.

### BRIEF DESCRIPTION OF THE DRAWINGS

FIG. 1 is a perspective view illustrating a multi-stimulation device according to an embodiment of the present invention.
FIG. 2 is an exploded view illustrating a stimulation unit of the multi-stimulation device.
FIG. 3 is a view illustrating a heat dissipation plate according to an embodiment of the present invention.
FIG. 4 is an exploded view illustrating a handle unit according to an embodiment of the present invention.
FIG. 5 is a view illustrating an item configuration for an experiment of the heat dissipation plate according to an embodiment of the present invention.
FIG. 6 is a view illustrating a structure of the heat dissipation plate of items used in an experimental example of FIG. 5.
FIG. 7 is a view illustrating a measurement state of experimental example of FIG. 5.

### MODE FOR CARRYING OUT THE INVENTION

The various embodiments described in this document are provided by way of example for the purpose of clearly illustrating the technical spirit of the present invention and disclosure, and are not intended to limit the invention to any particular embodiment. The technical spirit of the present invention and disclosure includes various modifications, equivalents, alternatives, and embodiments selectively combined from all or part of the embodiments described in this document. Moreover, the scope of rights of the technical spirit of the present invention and disclosure is not limited to the embodiments presented below or the detailed descriptions thereof.

Unless otherwise defined, the terms used in this document-including technical and scientific terms-shall be interpreted as having meanings commonly understood by those of ordinary skill in the art to which the present invention pertains.

Expressions such as "comprises," "may comprise," "includes," "may include," "has," and "may have," as used in this document, are intended to indicate the presence of stated features, functions, operations, or components, and do not preclude the presence or addition of one or more other features, functions, operations, or components. Accordingly, such expressions should be understood as open-ended terms that may encompass other embodiments.

The singular expressions used in this document may include the plural meaning as well, unless the context clearly dictates otherwise. This applies equally to the singular expressions recited in the claims.

The expressions used in this document such as "A, B, and C," "A, B, or C," "A, B, and/or C," "at least one of A, B, and C," "at least one of A, B, or C," "at least one of A, B, and/or C," "at least one selected from among A, B, and C," "at least one selected from among A, B, or C," and "at least one selected from among A, B, and/or C," are to be interpreted as encompassing all possible combinations of the listed elements or items. For example, the expression "at least one selected from among A and B" may refer to: (1) A; (2) at least one of A; (3) B; (4) at least one of B; (5) at least one of A and at least one of B; (6) at least one of A and B; (7) at least one of B and A; (8) both A and B.

The expression "based on" as used in this document is employed to describe one or more factors that influence a decision, judgment, operation, or action described in the phrase or sentence in which the expression appears, and does not exclude additional factors that may affect the decision, judgment, operation, or action.

Expressions used in this document stating that a certain component (for example, a first component) is "connected to" or "coupled with" another component (for example, a second component) may mean not only that the certain component is directly connected or coupled to the other component, but also that it is connected or coupled via an additional component (for example, a third component).

The expression "configured to" as used in this document may, depending on the context, be interpreted to mean "set to," "capable of," "adapted to," "modified to," "designed to," or "able to," and is to be distinguished from the expression "consists of."

Hereinafter, various embodiments of the present invention will be described with reference to the accompanying drawings. In the accompanying drawings and the descriptions thereof, the same or substantially equivalent components may be assigned the same reference numerals. Additionally, in the following descriptions of the various embodiments, repetitive explanations of the same or corresponding components may be omitted for the sake of clarity, but such omission does not imply that the components are not included in the respective embodiments.

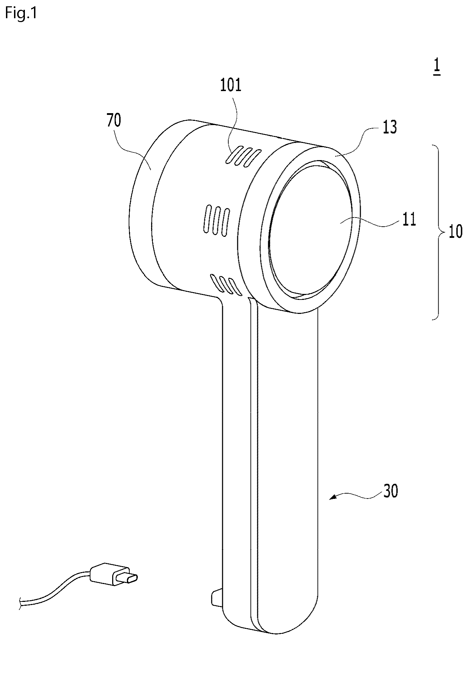

FIG. 1 is a perspective view illustrating a multi-stimulation device 1 according to an embodiment of the present invention.

Referring to FIG. 1, the multi-stimulation device 1 may be provided in the form of a housing of a handpiece, and the housing may include a stimulation unit 10 in which a first electrode 11 and a second electrode 13 are exposed and a handle unit 30. The multi-stimulation device 1 may include a first electrode 11, a second electrode 13, a thermoelectric element 20, a first heat dissipation plate 31, a second heat dissipation plate 33, a fan 70, and a control panel 60 as internal components.

The stimulation unit 10 may include the first electrode 11, the second electrode 13, the thermoelectric element 20, the first heat dissipation plate 31, the second heat dissipation plate 33, and the fan 70 to constitute a head of the housing. The stimulation unit 10 may perform all of medium-frequency stimulation, cold stimulation, and heat stimulation by a component to be described below. The stimulation unit 10 cools the thermoelectric element 20 by using an air-cooling method in which the fan 70 cools the heat dissipation plates 31and 33, and a ventilation hole 101 is formed in the housing in which the heat dissipation plates 31and 33 are disposed to quickly discharge heat generated from the heat dissipation plates 31 and 33 to the outside.

In this embodiment, the thermoelectric element 20 may include a thermistor that is an element using a temperature change of electrical resistance, an element using the Seebeck effect in which an electromotive force is generated due to a temperature difference, and a Peltier element using the Peltier effect in which heat is absorbed or generated due to electric current.

The first electrode 11 may have a predetermined surface area exposed to the outside to form a stimulation surface. The second electrode 13 may be spaced a distance from an outer circumferential surface of the first electrode 11. The second electrode 13 may be brought into contact with skin of a human body to serve as a ground electrode. The stimulation surface may include the first electrode 11 and the second electrode 13. In this embodiment, the first electrode 11 has a disc shape in which a predetermined positive curvature is formed at a central portion to form a convex stimulation surface at the central portion. The second electrode 13 has a circular band structure and is spaced outward from the first electrode 11 so that the second electrode 13 is not electrically connected to the first electrode 11. An insulating material may be inserted in a spaced space between the first electrode 11 and the second electrode 13. The stimulation surface formed by the first electrode 11 and the second electrode 13 allows the first electrode 11 and the second electrode 13 to be brought into contact with skin of a user through a curvature of the first electrode 11. The second electrode 13 is ground when brought into contact with the skin and forms a voltage difference from the first electrode 11 to apply a medium-frequency stimulation to a contacted skin surface. Also, the first electrode 11 may be changed in temperature due to the thermoelectric element 20 disposed on a rear surface, which will be described later, to simultaneously transmit cold stimulation or heat stimulation to the skin.

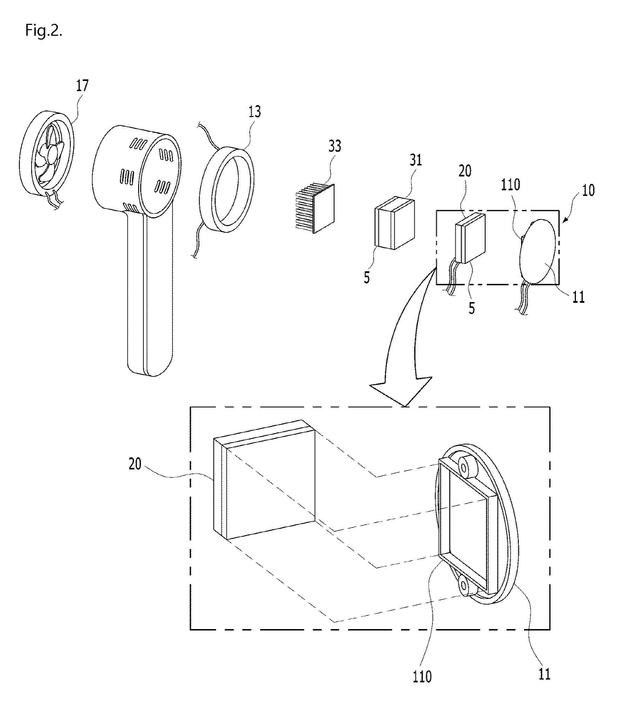

FIG. 2 is an exploded view illustrating the stimulation unit 10 of the multi-stimulation device 1. Referring to FIG. 2, when the stimulation surface faces a front surface, the first electrode 11, the thermoelectric element 20, the first heat dissipation plate 31, the second heat dissipation plate 33, and the fan 70 in the multi-stimulation device 1 according to the embodiment may be sequentially arranged on the same axis as the stimulation surface.

In the thermoelectric element 20, when one surface absorbs or generates heat due to applied electric current, the other surface may generate or absorb heat, and one surface may be brought into contact with a rear surface of the first electrode 11 to provide cold or heat.

It is noticeable in component arrangement of air-cooling heat exchanger according to the embodiment that the first electrode 11 is brought into contact with and coupled to a front surface of the thermoelectric element 20, and the first heat dissipation plate 31 and the second heat dissipation plate 33 are sequentially coupled to a rear surface thereof. For example, in a case in which the thermoelectric element 20 is a Peltier element, the rear surface absorbs heat when the heat is generated from the front surface of the thermoelectric element 20, and heat is generated from the rear surface when the front surface absorbs the heat. In general, since heat is generated from both surface of the thermoelectric element 20, a heat dissipation component is typically disposed on each of the front and rear surfaces. However, since the front surface of the thermoelectric element 20 is in direct contact with the skin of the user to transmit cold or heat depending on whether the front surface absorbs or generates heat in this embodiment, the embodiment of the present invention is limited to the heat dissipation component disposed on the rear surface of the thermoelectric element 20. More specifically, it is important to rapidly cool the rear surface of the thermoelectric element 20 when the rear surface generates heat, so that a heat absorption function of the rear surface of the thermoelectric element 20 is performed. This is because a heat absorption function of the front surface is smoothly performed as the cooled rear surface of the thermoelectric element 20 generates heat again when the thermoelectric element 20 is converted from heat stimulation into cold stimulation.

Accordingly, unlike a heat exchanger structure of a conventional thermoelectric element, the heat dissipation structure may be disposed on only one surface, i.e., the rear surface, due to a design constraint instead of being disposed on each of the front and rear surfaces of the thermoelectric element 20 according to the embodiment. Thus, a performance of air cooling at the rear surface determines whether the device capable of simultaneously performing cold and heat stimulation is implemented.

Hereinafter, the heat dissipation structure according to the embodiment will be described in detail. A coupling part 110 may be disposed on the rear surface of the first electrode 11. The coupling part 110 is a groove for accommodating the thermoelectric element 20, and the thermoelectric element 20 is fitted to the coupling part 110. Here, the coupling part 110 may be a groove formed by partition walls to contact not only the front surface but also side surfaces of the thermoelectric element 20. The thermoelectric element 20 may be brought into contact and coupled with the first electrode 11 through the coupling part 110, and a compound layer 5 may be disposed on the front surface of the thermoelectric element 20, which is fitted into and in contact with the first electrode 11. In this embodiment, the compound layer 5 may be disposed on a contact surface, in which the first heat dissipation plate 31 and the second heat dissipation plate 33 are coupled, to improve a cooling efficiency of the heat dissipation plates arranged in a single direction in the multi-stimulation device 1. Also, the compound layer 5 may be disposed on a contact surface in which the first electrode 11 and the thermoelectric element 20 are coupled.

In this embodiment, the compound layer 5 that is formed by applying a viscous liquid may include thermal grease, thermally conductive paste, thermally conductive silicone, or a thermally conductive composite.

The compound layer 5 increases thermal conductivity due to a property of a material thereof. Here, it is noticeable that a compound is applied in the form of a viscous liquid. This aspect allows the compound layer 5 to fill micro-non-contact pores between coupling surfaces of metals to further improve thermal conductivity between the metals.

In this embodiment, the heat dissipation plates 31 and 33 includes two pieces of the first heat dissipation plate 31 and the second heat dissipation plate 33. The thermoelectric element 20 has a relatively thin plate shape due to characteristics thereof. Accordingly, the thermoelectric element 20 has a low heat capacity due to characteristics of a material thereof. In this embodiment, the heat dissipation plates are provided in the form of two pieces to compensate for the low heat capacity of the thermoelectric element 20. Firstly, the first heat dissipation plate 31 is formed of a block-shaped metal material to have a large volume and a high heat capacity. Specifically, the first heat dissipation plate 31 firstly accommodates heat to prevent the thermoelectric element 20 from being rapidly heated, and then the second heat dissipation plate 33 cools the heat with a large surface area thereof.

Here, it is noticeable that the compound layer 5 is formed between the first heat dissipation plate 31 and the second heat dissipation plate 33. The compound layer 5 fills micro-uneven portions or pores in metal coupling surfaces of the first heat dissipation plate 31 and the second heat dissipation plate 33 with a viscous liquid to ensure complete surface adhesion when the metal materials of the first heat dissipation plate 31 and the second heat dissipation plate 33 are coupled, thereby significantly improving the thermal conductivity between the metals.

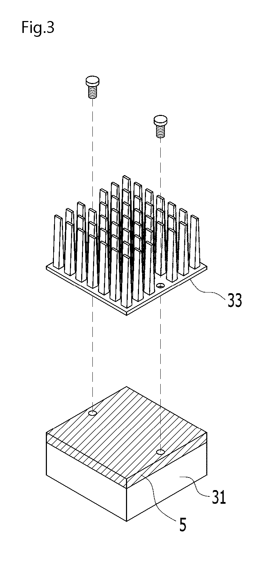

FIG. 3 is a view illustrating the heat dissipation plates 31and 33 according to an embodiment of the present invention.

Referring to FIG. 3, the first heat dissipation plate 31 may be brought into contact with the other surface of the thermoelectric element 20 and have a block shape having a predetermined thickness. The compound layer 5 may be applied to an upper portion of the first heat dissipation plate 31. Also, a screw hole for coupling with the second heat dissipation plate 33 may be defined in the upper portion of the first heat dissipation plate 31.

The second heat dissipation plate 33 may be coupled to the first heat dissipation plate 31, and a fin array having a surface area for dissipating heat into the atmosphere may be formed on the second heat dissipation plate 33. The fin array may include a plurality of plate-shaped rods on which uneven portions are formed to provide a large surface area. A screw hole may be defined in the second heat dissipation plate 33 for being coupled with the first heat dissipation plate 31. The first heat dissipation plate 31 and the second heat dissipation plate 33 may be made of a metal material having high thermal conductivity and coupled using a screw. In an assembly process of the first heat dissipation plate 31 and the second heat dissipation plate 33, a completely tight contact state between contact surfaces of the first heat dissipation plate 31 and the second heat dissipation plate 33 may not be implemented due to characteristics of metal machining although the two metals are coupled using the screw with extremely strong force. Also, when a plurality of screw holes are defined in the first heat dissipation plate 31 and the second heat dissipation plate 33 to further strengthen the tight contact state therebetween, the fin array is not sufficiently formed on the second heat dissipation plate 33, and thus a cooling efficiency is not secured. Thus, since the coupling between the first heat dissipation plate 31 and the second heat dissipation plate 33 is a key design matter for securing the cooling efficiency, the compound layer 5 is formed in this embodiment to increase thermal conductivity and achieve the tight contact state therebetween.

On the other hand, when a stimulation surface of the second heat dissipation plate 33 faces the front surface, the second heat dissipation plate 33 may be arranged so that a direction in which pins of the fin array protrude faces the rear surface. The fan 70 disposed behind the head part may generate a flow of air toward the second heat dissipation plate 33. Since the above-described embodiment of the configuration of the heat exchanger achieves the sufficient thermal conductivity and cooling efficiency, the cold and heat stimulation may be implemented although the pin array of the second heat dissipation plate 33 is not arranged in vertical both directions and a longitudinal direction based on a product illustrated in FIG. 1. As a result, in the heat-exchange configuration according to the embodiment, the second heat dissipation plate 33 may face the rear surface based on the product in FIG. 1, and air-cooling may be performed directly from the fan 70 disposed on the rear to form a preferred assembly structure in terms of the cooling efficiency. Also, as the second heat dissipation plate 33 is arranged in a direction toward the rear surface, and the stimulation unit 10 is shortened in a front and rear direction based on the product in FIG. 1, a center of gravity may be stabilized, and a usage feeling of the user may be improved.

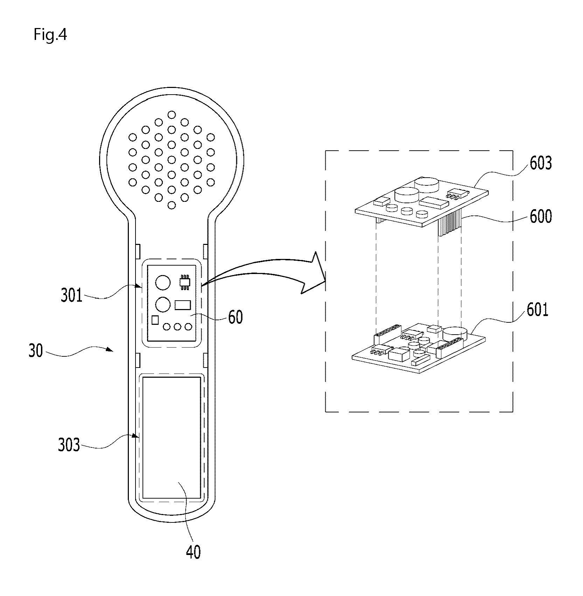

FIG. 4 is an exploded view illustrating the handle unit 30 according to an embodiment of the present invention.

The control panel 60 may be disposed on the handle unit 30, and the handle unit 30 may include a rechargeable battery 40 that supplies electrical power to the control panel. The control panel 60 that is a circuit component for controlling the thermoelectric element 20 or the first electrode 11 may control the medium-frequency stimulation caused by the first electrode 11 or the heat absorption or heat generation of the thermoelectric element 20 to form the cold or heat stimulation on a stimulation surface.

Referring to FIG. 4, the handle unit 30 is divided into a first section 301 and a second section 303. The control panel 60 may be disposed in the first section 301, and the battery 40 may be disposed in the second section 303.

The control panel 60 may have a stacked structure in which a first control panel 601 and a second control panel 603 are stacked and be disposed in the first section 301. In an embodiment, a medium-frequency module may be mounted to the first control panel 601, and a power supply module for controlling the thermoelectric element 20 may be mounted to the second control panel 603.

The connection part 600 may be formed for stacked coupling between the first control panel 601 and the second control panel 603. The connection part 600 may be implemented as a pin for power supply, and the second control panel 603 may receive power supplied to the first control panel 601 through the connection part 600.

### < Experimental example of heat dissipation plate - Performance test of heat dissipation plate >

Hereinafter, a performance test of the first heat dissipation plate 31 and the second heat dissipation plate 33 according to the embodiment of the present invention disclosed in FIG. 3 will be described. The disclosed first heat dissipation plate 31 and second heat dissipation plate 33 are compared with a heat dissipation structure of a first-generation product disclosed in Korean Patent Registration No. 1193935 that is a related art of the present applicant.

Through this experimental example, it is confirmed that a thermal/cooling efficiency of a two-piece structure of the heat dissipation plate according to the embodiment is varied based on whether the compound layer is present. Hereinafter, the experimental example will be described in detail.

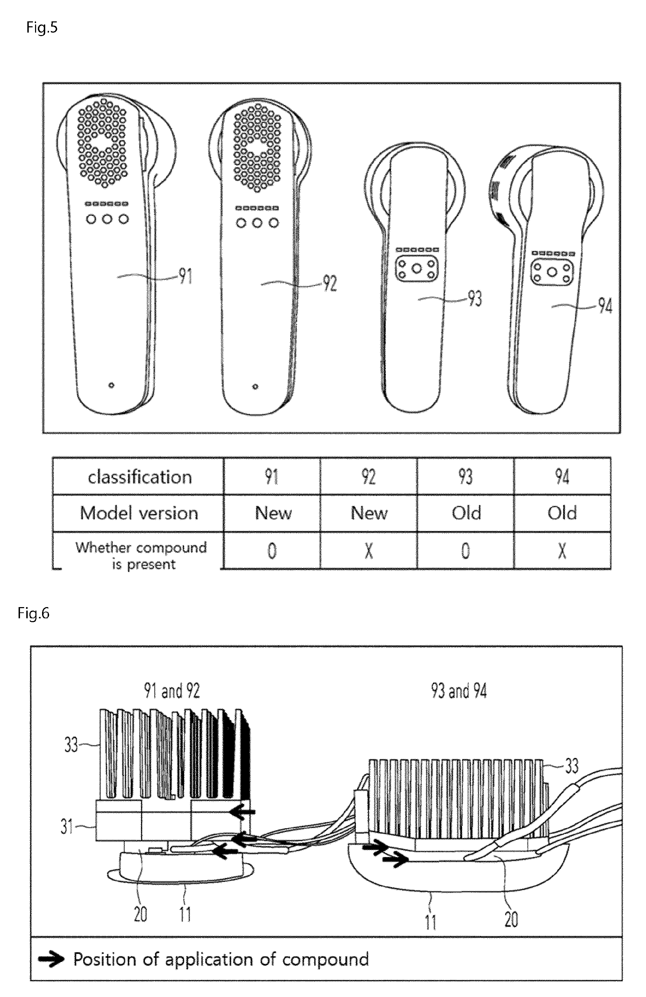

FIG. 5 a view illustrating an item configuration for the experiment of the heat dissipation plates 31 and 33 according to an embodiment of the present invention. FIG. 6 is a view illustrating the structure of the heat dissipation plate of the item used in the experimental example of FIG. 5. Referring to FIGS. 5 and 6, each of test items 1 and 2 uses two-piece heat dissipation module including the first heat dissipation plate 31 and the second heat dissipation plate 33 according to the above-described embodiment of FIG. 3. Hereinafter, each of the test items 1 and 2 will be referred to as a 'new model'. Although each of the test item 1 and the test item 2 includes the first heat dissipation plate 31 and the second heat dissipation plate 33, the test item 1 includes a module with the compound layer 5, while the test item 2 includes a module without the compound layer 5.

Each of a test item 3 and a test item 4 that are related products of the present applicant of Korean Patent Registration No. 1193935 uses a heat dissipation module including a single heat dissipation plate. Hereinafter, each of the test item 3 and the test item 4 will be referred to as an 'old model'. Although each of the test item 3 and the test item 4 includes the same single heat dissipation module, the test item 3 includes a module in which the compound layer 5 is formed at a lower end, while the test item 4 includes a module in which the compound layer 5 is not formed.

FIG. 7 is a view illustrating a measurement state of the experimental example.

A test method will be described below.

It is checked whether an environment is the same as an initial experiment environment by checking an ambient temperature, and then, a tip of a thermometer is connected to an electrode ceramic of the test item. A surface temperature of the electrode ceramic of the test item is measured before operation, and then, a cooling mode and a heating mode of the test item are activated. A variation in surface temperature of the electrode ceramic is measured at 10-second intervals for up to 2 minutes by using a stopwatch. Each test item is measured one time in cooling mode and one time in heating mode. A measurement condition is shown in [Table 1].

**[Table 1]**

| Classification | Test item 1 | Test item 2 | Test item 3 | Test item 4 |
|---|---|---|---|---|
| Model version | New | New | Old | Old |
| Compound | O | X | O | X |
| Ambient temperature (°C) | 23.5 | 23.7 | 24 | 23.9 |
| Electrode surface temperature (°C) | 22.6 | 22.4 | 22.6 | 23.0 |

Performance is determined as follows.

In the cooling mode, the performance is determined based on an order of a fastest time required for the surface temperature of the electrode ceramic to decrease. In the heating mode, the performance is determined based on an order of a fastest time required for the surface temperature of the electrode ceramic to increase.

Test results are as follows.

[Table 2] shows results of the cooling performance measurement test.

**[Table 2]**

| Classification | Test item 1 | Test item 2 | Test item 3 | Test item 4 |
|---|---|---|---|---|
| Model version | New | New | Old | Old |
| Compound | O | X | O | X |
| Time(min/sec) | Electrode surface temperature (°C) | | | |
| 0/0 | 22.6 | 22.4 | 22.6 | 23.0 |
| 0/10 | 19.0 | 21.9 | 20.2 | 20.6 |
| 0/20 | 15.3 | 20.7 | 17.9 | 18.1 |
| 0/30 | 12.0 | 19.6 | 15.9 | 16.2 |
| 0/40 | 9.2 | 18.4 | 14.3 | 14.8 |
| 0/50 | 7.3 | 17.5 | 13.2 | 13.6 |
| 1/00 | 5.9 | 16.6 | 12.2 | 12.6 |
| 1/10 | 4.7 | 16.0 | 11.3 | 11.9 |
| 1/20 | 4.1 | 15.4 | 10.6 | 11.3 |
| 1/30 | 3.7 | 14.9 | 9.9 | 10.8 |
| 1/40 | 3.4 | 14.6 | 9.5 | 10.5 |
| 1/50 | 3.2 | 14.3 | 9.2 | 10.2 |
| 2/00 | 3.0 | 14.0 | 8.9 | 9.9 |

Referring to [Table 2], the results of the cooling performance test are evaluated in an order: Test item 1 > Test item 3 > Test item 4 > Test item 2. According to the test results, it is confirmed that the compound layer 5 gives a greater effect on cooling performance than the two-piece structure of the heat dissipation plate. Next, when the compound layer 5 is not present, it is confirmed that the heat dissipation module including the two-piece of the first heat dissipation plate 31 and the second heat dissipation plate 33 exhibits excellent cooling performance.

When the compound layer 5 is present in both cases, Test item 1 including the first heat dissipation plate 31 and the second heat dissipation plate 33 exhibits a temperature difference of 5.9°C in cooling performance from Test item 3 including a single heat dissipation plate.

Also, Test item 1 including the compound layer 5 and including the first heat dissipation plate 31 and second heat dissipation plate 33 exhibits a temperature difference of 6.9°C in cooling performance from Test item 4 including a conventional heat dissipation module.

[Table 3] shows results of a heating performance measurement test.

**[Table 3]**

| Classification | Test item 1 | Test item 2 | Test item 3 | Test item 4 |
|---|---|---|---|---|
| Model version | New | New | Old | Old |
| Compound | O | X | O | X |
| Time(min/sec) | Electrode surface temperature (°C) | | | |
| 0/0 | 22.8 | 22.8 | 22.7 | 22.6 |
| 0/10 | 24.7 | 23.2 | 24.9 | 23.9 |
| 0/20 | 27.0 | 24.0 | 26.4 | 25.9 |
| 0/30 | 28.7 | 24.8 | 28.2 | 27.4 |
| 0/40 | 30.5 | 25.7 | 29.2 | 29.0 |
| 0/50 | 31.9 | 26.5 | 30.5 | 30.2 |
| 1/00 | 33.3 | 27.3 | 31.7 | 31.5 |
| 1/10 | 34.4 | 28.1 | 33.0 | 32.3 |
| 1/20 | 35.2 | 28.8 | 33.4 | 33.5 |
| 1/30 | 36.0 | 29.5 | 34.3 | 34.3 |
| 1/40 | 35.9 | 30.1 | 34.8 | 35.1 |
| 1/50 | 36.1 | 30.7 | *35.5* | 35.8 |
| 2/00 | 36.1 | 31.3 | 36.2 | 36.3 |

Referring to [Table 3], the heating performance test exhibits generally similar results with no significant variation in temperature differences. However, it is confirmed in this test that whether the compound layer 5 is present in the two-piece heat dissipation plate structure exhibits a meaningful temperature difference of about 5°C.

While the technical spirit of the present invention and disclosure has been described with reference to the above embodiments, it should be understood that the technical scope of the present invention includes various substitutions, modifications, and alterations that may be made by those skilled in the art without departing from the scope of the invention. Such substitutions, modifications, and alterations are to be understood as falling within the scope of the appended claims.

1: Multi-stimulation device
5: Compound
10: Stimulation unit
11: First electrode
13: Second electrode
20: Thermoelectric element
30: Handle unit
31: First heat dissipation plate
33: Second heat dissipation plate
110: Coupling part
101: Ventilation hole
301: First section
303: Second section
40: Battery
60: Control panel
600: Connection part
601: First control panel
603: Second control panel
70: Fan

### INDUSTRIAL APPLICABILITY

The present invention may significantly improve muscle pain relief and recovery rate by using multi-stimulation of the cold and heat stimulation and the medium-frequency stimulation and thus be effectively used in physical therapy.

## Claims

1. A multi-stimulation device for pain management and physical therapy, the multi-stimulation device comprising:
a first electrode having a predetermined surface area exposed to the outside to form a stimulation surface;
a thermoelectric element in which, when one surface absorbs or generates heat by applied electric current, the other surface generates or absorbs heat, and one surface is brought into contact with a rear surface of the first electrode to provide cold or heat;
a first heat dissipation plate being in contact with the other surface of the thermoelectric element and having a block shape with a predetermined thickness;
a second heat dissipation plate coupled to the first heat dissipation plate and comprising a fin array having a surface area for dissipating heat into the atmosphere;
a fan configured to generate a flow of air to the second heat dissipation plate; and
a control panel configured to control the thermoelectric element or the first electrode, thereby controlling medium-frequency stimulation of the first electrode or heat absorption or heat generation of the thermoelectric element and forming the cold or heat stimulation on the stimulation surface,
wherein the second heat dissipation plate is arranged such that, when the stimulation surface faces a front surface of the device, fins of the fin array protrude toward a rear surface of the device.

2. The multi-stimulation device of claim 1, wherein, when the stimulation surface faces the front surface, the first electrode, the thermoelectric element, the first heat dissipation plate, the second heat dissipation plate, and the fan are sequentially arranged on the same axis as the stimulation surface.

3. The multi-stimulation device of claim 1, wherein a compound layer is disposed on a contact surface in which the first heat dissipation plate and the second heat dissipation plate are coupled.

4. The multi-stimulation device of claim 1, wherein a compound layer is disposed on a contact surface in which the first electrode and the thermoelectric element are coupled.

5. The multi-stimulation device of claim 3 or 4, wherein the compound layer that is formed by applying a viscous liquid comprises thermal grease, thermally conductive paste, thermally conductive silicon, or a thermally conductive composite.

6. The multi-stimulation device of claim 1, further comprising a second electrode spaced a distance from an outer circumferential surface of the first electrode,
wherein the second electrode is brought into contact with skin of a human body to serve as a ground electrode, and
the stimulation surface is formed by the first electrode and the second electrode.

7. The multi-stimulation device of claim 1, wherein the first electrode, the thermoelectric element, the first heat dissipation plate, the second heat dissipation plate, and the fan are disposed in the stimulation unit that constitutes a head of the housing,
the control panel is disposed in the handle unit of the housing, and
the handle unit comprises a rechargeable battery configured to supply power to the control panel.

8. The multi-stimulation device of claim 7, wherein the handle unit is divided into a first section and a second section,
the control panel is disposed in the first section, and
the battery is disposed in the second section.

9. The multi-stimulation device of claim 8, wherein the control panel has a stacked structure in which a first control panel and a second control panel are stacked and is disposed in the first section.
